(19) Europäisches Patentamt
European Patent Office
Office européen des brevets

(11) Veröffentlichungsnummer: **0 009 214**
**B1**

(12) # EUROPÄISCHE PATENTSCHRIFT

(45) Veröffentlichungstag der Patentschrift: **08.09.82**

(51) Int. Cl.³: **G 01 N 23/222,** G 01 N 33/12

(21) Anmeldenummer: **79103434.1**

(22) Anmeldetag: **14.09.79**

(54) **Strahlungsmessverfahren und -vorrichtung für Konzentrationsbestimmungen von Fett in Fleischmassen.**

(30) Priorität: **22.09.78 DE 2841307**

(43) Veröffentlichungstag der Anmeldung: **02.04.80 Patentblatt 80/7**

(45) Bekanntmachung des Hinweises auf die Patenterteilung: **08.09.82 Patentblatt 82/36**

(84) Benannte Vertragsstaaten: **BE FR GB NL SE**

(56) Entgegenhaltungen:
**US-A-2 992 332**
**Isotopes and Radiation Technology, Band 9, Nr. 3, Frühling 1972, Seiten 351-353**
**Oak Ridge, U.S.A.**
**D.E. WOOD: "Protein Determination by Neutron Activation Analysis"**

(73) Patentinhaber: **Kernforschungsanlage Jülich Gesellschaft mit beschränkter Haftung, Postfach 1913, D-5170 Jülich (DE)**

(72) Erfinder: **Kasperek, Karl, Mozartweg 5, D-5160 Düren (DE)**
Erfinder: **Glozbach, Peter, Dr., Wiesenstrasse 1, D-5170 Jülich (DE)**

**EP 0 009 214 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

Strahlungsmessverfahren und -vorrichtung für Konzentrationsbestimmungen von Fett in Fleischmassen

Die Erfindung bezieht sich auf ein Strahlungsmessverfahren zur zerstörungsfreien Ermittlung des Fettgehalts von Fleischmassen sowie auf Vorrichtungen zur Durchführung des Verfahrens.

Die Überwachung des Fettgehalts von Fleisch- und Wurstwaren in fleischverarbeitenden Betrieben setzt eine den Produktionsablauf möglichst wenig störende und rasche Fettbestimmungsmethode voraus. Bekannt sind Messungen über das spezifische Gewicht beziehungsweise über den Auftrieb, Absorptionsmessungen mit Röntgenstrahlen sowie chemische Verfahren, bei denen der Fettanteil nach einer Infrarot-Trocknung auf chemischem Wege bestimmt wird oder die Fettbestimmung nach einem Säureaufschluss über die Messung des Brechungsindex erfolgt. Ein Röntgenabsorptionsverfahren zur Fettbestimmung in Fleischproben ist z.B. in der Patentschrift US-A-2 992 332 beschrieben.

Bei allen bekannten Verfahren ist weder eine schnelle noch eine kontinuierliche Fettbestimmung möglich. Die Röntgenabsorptionsmessung ist zwar zerstörungsfrei, sie wird aber nur an entnommenen Proben durchgeführt, und der Produktionsprozess muss unterbrochen werden. Bei allen Bestimmungen werden nur mehr oder minder kleine Proben untersucht, die meistens im Gramm-Bereich liegen. Lediglich bei der Fettbestimmung über das spezifische Gewicht werden 17 kg ausgemessen.

Der Erfindung liegt daher die Aufgabe zugrunde, eine möglichst kontinuierliche Fettbestimmung ohne Zeitverlust unter Erfassung grösserer Anteile der Produktion zu schaffen.

Das zur Lösung dieser Aufgabe entwickelte Strahlungsmessverfahren basiert auf der Erfassung der durch Neutronen ausgelösten prompten $\gamma$-Strahlung, die - insbesondere bei diskriminierender Erfassung - über entsprechende Eichkurven unmittelbare Konzentrationsanzeigen zulässt.

Das erfindungsgemässe Strahlungsmessverfahren der eingangs genannten Art ist demgemäss dadurch gekennzeichnet, dass man die Fleischmasse der Neutronenstahlung einer n-Quelle aussetzt und die durch $(n,\gamma)$-Reaktion ausgesandte, für C allein oder die Elemente C, H und N elementspezifische prompte $\gamma$-Strahlung selektiv nachweist und die dafür ermittelte(n) Intensität(en) mit entsprechenden Eichkurven vergleicht bzw. in Fettgehalt umrechnet.

Vorrichtungen zur Durchführung des Verfahrens sind in den Ansprüchen 5 und 6 gekennzeichnet. Zusätzlich kann auch die aus einer Aktivierung resultierende verzögerte $\gamma$-Strahlung gemessen werden, wie zum Beispiel die aus den Reaktionen

$$(1)\ \text{C-12}\,(n,2n)\,\text{C-11} \xrightarrow[20{,}3\ \text{min}]{\beta^+} \text{B-11}$$

oder

$$(2)\ \text{O-16}\,(n,p)\,\text{N-16} \xrightarrow[7{,}4\ \text{sec}]{\beta^-,\gamma} \text{O-16}$$

resultierende Strahlung.

Diese kann durch eine zusätzliche Messtelle erfasst werden, die auf dem Transportweg in einem geeigneten Abstand von der Bestrahlungsstelle vorgesehen wird.

Diese zusätzliche Erfassung der von einer n-Aktivierung resultierenden verzögerten Strahlung entspricht praktisch einem Messverfahren wie es aus «Isotopes and Radiation Technology», Band 9 Nr. 3, 1972, Seiten 351 bis 353 bekannt ist. Hier wird von D.E. Wood eine Proteinbestimmung in Nahrungsmitteln aufgrund des darin allein vorhandenen Stickstoffes beschrieben, der nach der Gleichung $^{14}N(n,2n)^{13}N$ aktiviert wird und als $^{13}N$ mit einer Halbwertzeit von 10 min $\gamma$-Strahlung von 0,51 MeV emittiert. Dieser Nachweis erfolgt unter Heranziehung eines nur für den zu bestimmenden Stoff spezifischen Elements und mit einer erheblich höheren n-Strahlenbelastung des zu untersuchenden Materials als bei einer Messwertermittlung über die prompte $\gamma$-Strahlung.

Bei der erfindungsgemässen Fettbestimmung in Fleischmassen wird primär die durch Neutroneneinstrahlung ausgelöste prompte $\gamma$-Strahlung gemessen, die im wesentlichen von den folgenden Reaktionen stammt:

C-12 $(n,\gamma1)$ C-13, insbesondere $\gamma1 = 1{,}26$ MeV

H-1 $(n,\gamma2)$ H-2, insbesondere $\gamma2 = 2{,}23$ MeV

N-14 $(n,\gamma3)$ N-15, insbesondere $\gamma3 = 5{,}3$ MeV.

Andere charakteristische prompte $\gamma$-Emissionen liegen zum Beispiel bei 4,95 MeV für C und bei 1,88 MeV für N.

Diese Reaktionen erfolgen insbesondere mit thermischen Neutronen, wobei jedoch zur n-Einstrahlung - wegen der grösseren Reichweite - im allgemeinen schnelle Neutronen verwendet werden, wie sie zum Beispiel von Cf-252 ausgestrahlt werden.

Die Strahlungsmessung führt über eine Umrechnung wie nachfolgend skizziert beziehungsweise unter Zugrundelegung von Eichkurven zur Konzentrationsangabe.

Wie aus der nachfolgenden Tabelle hervorgeht, haben die wesentlichen Komponenten von knochenfreiem Fleisch, das heisst Wasser, Eiweiss und Fett, deutlich unterschiedliche (angenäherte globale) Elementarzusammensetzungen, die unter Vernachlässigung der nur geringen Anteile an Kohlehydraten, Nukleinsäuren und Spurenelementen in knochenfreiem Fleisch eine relativ einfache Bestimmung des Fettgehalts gestatten.

| Komponenten | üblicher Anteil in knochenfreiem Fleisch % | Prozentualer Anteil der Elemente im Molekül % | Prozentualer Anteil der auf die einzelnen Komponenten zurückgehenden Elemente im Fleisch % |
|---|---|---|---|
| Wasser (W) | 60% | 11% H | 6,6%$H_W$ |
| | | 89% O | 53,4%$O_W$ |
| Eiweiss (E) | 20% | 16% N | 3,2%$N_E$ |
| | | 43% C | 8,6%$C_E$ |
| | | 41% O,H,[P,S] | 8,2%$O_E$,$H_E$[P,S] |
| Fett (F) | 15% | 76% C | 11,4%$C_F$ |
| | | 24% O,H | 3,6%$O_F$,$H_F$ |
| Kohlehydrate | 1% | 40% C | 0,4%C |
| | | 60% O,H | 0,6%O,H |
| Nukleinsäuren | 1% | 40% C | 0,4%C |
| | | 60% O,H,N,P,S | 0,6%O,H,N,P,S |
| Elektrolyte und Spurenelemente | 3% | | 3,0% |
| | 100% | | 100% |

Setzt man vereinfachend einen gleichbleibenden Wassergehalt voraus, so kann bereits aus der Bestimmung des (beim Fett mit 76% deutlich höher als beim nur 43% C aufweisenden Eiweiss liegenden) C-Gehalts der Masse durch diskriminierende Erfassung der γ-Strahlung bei 1,26 MeV rechnerisch beziehungsweise unter Zugrundelegung von Eichkurven auf den enthaltenen Fettanteil geschlossen werden.

Vorzugsweise wird jedoch die entstehende prompte γ-Strahlung mit einem 3-Kanal-Analysator erfasst, so dass die auf C(1,26 MeV), H(2,23 MeV) und N(5,3 MeV) zurückgehenden Anteile gesondert erhalten werden.

Lässt man zunächst den Wasseranteil ausser Acht, so gilt für den Fettgehalt der Masse folgende Gleichung:

$$\% \text{ Fett} \simeq 1{,}32\,[\% \text{ C gesamt} - \% \text{ C Eiweiss}]$$

$$\simeq 1{,}32\,[\% \text{ C gesamt} - 2{,}7\,\% \text{ N Eiweiss}]$$

$$\simeq 1{,}32\,\% \text{C gesamt} - 3{,}6\,\% \text{ N Eiweiss}$$

$$\simeq 1{,}32\, F_C \text{ Akt (C)} - 3{,}6\, F_N \text{ Akt (N)}$$

wobei $F_C$ und $F_N$ Faktoren für die Umrechnung der ermittelten Strahlungsmesswerte für C beziehungsweise N [Akt (C) beziehungsweise Akt (N)] in die entsprechenden C- beziehungsweise N-Gehalte sind und vereinfachend vorausgesetzt wird, dass der in der Masse enthaltene Stickstoff lediglich vom Eiweiss stammt.

Der Wasseranteil kann, ausgehend von der Gleichung:

$$\% H_2O \simeq K \cdot [\text{H gesamt} - 0{,}44\, N_E - 0{,}15\, C_F]$$

berücksichtigt werden, von der ausgehend Korrekturen bei der Ermittlung des Fettgehalts anzubringen wären (da ein erhöhter Wassergehalt einen geringeren Messwert für den Gesamtkohlenstoff ergibt, was wiederum einen geringeren Fettanteil vortäuscht).

Nachfolgend wird eine einfache Vorrichtung zur Bestimmung des Fettgehalts in Fleischmassen beschrieben, die in fleischverarbeitenden Betrieben eingesetzt werden kann.

Die nachfolgende Beschreibung bezieht sich auf die angefügte Zeichnung, in der schematisch ein Transportwagen mit Messgut und Neutronenquelle dargestellt und zusätzlich Detektoren und Messeinrichtungen angedeutet sind.

Die wesentlichen Elemente der erfindungsgemässen Vorrichtung sind die n-Quelle 1, die im vorliegenden Fall durch 1μg Cf-252 gebildet wird sowie die zur Aufnahme der prompten γ-Strahlung dienenden Detektoren 2 und 3, die an einen als 3-Kanal-Analysator 4 ausgebildeten Diskriminator mit nachfolgendem Kleinrechner 5 angeschlossen sind.

Im Normwagen 6 befindet sich die Fleischmasse 7, in welche die n-Quelle vorzugsweise eintaucht, wodurch Messfehler durch unterschiedliche Füllhöhen der Transportwagen vermieden werden können. Der von der Messung erfasste Bereich ist mit 8 bezeichnet.

Eine besonders einfache Konstruktion ergibt sich, wenn man die Transportwagen für die Fleichmasse in der zeichnerisch angedeuteten Weise mit einem stirnseitig offenen Innentrog 9 versieht, der mechanisch durch eine Verbindung 10 zum Boden hin zusätzlich unterstützt sein kann (oder gegebenenfalls nach unten weisend einen Bodentunnel bildet), und im Transportweg des Wagens eine ortsfeste n-Quelle 1 anordnet. Man kann jedoch auch in einer bestehenden Produktionsanlage eine Messtelle vorsehen, die eine in die Fleischmasse selbst versenkbare beziehungsweise einfahrbare n-Quelle 1 mit entsprechender Hülle umfasst, die entweder kurzzeitig in den stehenden Wagen eingebracht und wieder herausgezogen wird oder in den langsam vorrückenden Transportwagen einfährt und in diesem relativ

zum Gut vom vorderen zum hinteren Ende bewegt wird.

Als Strahlungsdetektoren können Szintillationszähler mit nachgeschaltetem Diskriminator dienen. Die n-Strahlenquelle kann, wie bereits angedeutet wurde, zum Beispiel durch 1µg Cf-252 gebildet werden. Der vor einer solchen Quelle ausgehende Neutronenstrom liegt in der Grössenordnung von $10^6$ bis $10^7$ n/s und ist damit hinreichend gering, so dass keine wesentlichen Aktivierungen des Gutes zu befürchten sind. Die entstehende höchste Radioaktivität von Radioisotopen mit Halbwertszeiten über 1 min dürfte vom C1-38 stammen und etwa in der Gegend von 2 nC/kg liegen, also bei nur etwa 1/50 der im Organismus ohnehin vorhandenen Aktivität von K-40. Die Halbwertszeit von C1-38 liegt bei 37 min, so dass nach 6 Stunden nur von 1/1000 und nach weiteren 6 Stunden 1 Millionstel der Aktivität vorhanden ist, das heisst, die erzeugte Radioaktivität ist abgeklungen.

**Patentansprüche**

1. Strahlungsmessverfahren zur zerstörungsfreien Ermittlung des Fettgehalts von Fleischmassen, dadurch gekennzeichnet, dass man die Fleischmasse der Neutronenstrahlung einer n-Quelle aussetzt und die durch (n,γ)-Reaktion ausgesandte, für C allein oder die Elemente C, H und N elementspezifische prompte γ-Strahlung selektiv nachweist und die dafür ermittelte(n) Intensität(en) mit entsprechenden Eichkurven vergleicht bzw. in Fettgehalt umrechnet.

2. Verfahren nach Anspruch 1, dadurch gekennzeichnet, dass man die prompte γ-Strahlung bei 1,26 MeV misst.

3. Verfahren nach Anspruch 2, dadurch gekennzeichnet, dass man zusätzlich die γ-Strahlung bei 2,23 und 5,3 MeV misst.

4. Verfahren nach Anspruch 1, dadurch gekennzeichnet, dass man zusätzlich die verzögerte γ-Strahlung von gebildeten Aktivierungsprodukten aufnimmt.

5. Vorrichtung zur Durchführung des Verfahrens nach Anspruch 1, gekennzeichnet durch eine in einen Fleischtransportweg einzubringende Messtelle mit einer so angeordneten n-Strahlenquelle, dass die passierenden Fleischmassen ihrer Strahlung ausgesetzt sind, sowie zumindest mit einem γ-Strahlendetektor (2;3) mit angeschlossenem Diskriminator (4) und gegebenenfalls mit einem Rechner (5).

6. Vorrichtung zur Bestimmung des Fettgehalts von Fleischmassen nach Anspruch 1, gekennzeichnet durch zumindest einen Fleischtransportwagen (6) mit einem stirnseitig offenen Innentrog (9) oder Tunnel und durch eine Messstelle im Transportweg der Fleischmasse mit einer Neutronenquelle (1), die in den Trog oder Tunnel eingreift, wenn der Wagen die Messtelle passiert, sowie durch neben dem Transportwagen beziehungsweise ausserhalb desselben angeordneten γ-Detektoren (2, 3) mit zugeordnetem Analysator (4) und gegebenenfalls mit einem Rechner (5).

**Claims**

1. Radiation-measuring process for the nondestructive determination of the fat content of mixtures or masses of meat, characterised in that the meat mixture is exposed to the neutron radiation of an n-source and the prompt γ-radiation emitted as a result of (n,γ) reaction and specific for C alone or for the elements C, H and N is detected selectively, and the intensity or intensities determined for this are compered with appropriate calibration curves or are converted into a fat content.

2. Process according to Claim 1, characterised in that the prompt γ-radiation is measured at 1.26 MeV.

3. Process according to Claim 2, characterised in that the γ-radiation is additionally measured at 2.23 and 5.3 MeV.

4. Process according to Claim 1, characterised in that the delayed γ-radiation from activation products formed is also detected.

5. Apparatus for carrying out the process according to Claim 1, characterised by a measuring point to be introduced into a meat transport path, with an n-ray source which is arranged so that the passing meat mixtures are exposed to its radiation, and with at least one γ-ray detector (2, 3) with a connected discriminator (4) and, if appropriate, with a computer (5).

6. Apparatus for determining the fat content of meat mixtures according to Claim 1, characterised by at least one meat transport truck (6) with an inner trough (9) open on the end faces, or tunnel, and by a measuring point in the transport path of the meat mixture, with a neutron source (1) which engages into the trough or tunnel when the truck passes the measuring point, and by γ-detectors (2, 3) located next to the transport truck and outside the latter, with an associated analyser (4) and, if appropriate, with a computer (5).

**Revendications**

1. Procédé de mesure de rayonnement pour la détermination, sans destruction, de la teneur en graisse de masses de viande, caractérisé en ce qu'on expose la masse de viande au rayonnement neutronique d'une source n et on décèle sélectivement le rayonnement γ instantané émis par la réaction (n, γ) et spécifique à C uniquement ou aux éléments C, H et N, puis on compare la ou les intensité(s) déterminée(s) à cet effet avec des courbes d'étalonnage correspondantes ou on les convertit en une teneur en graisse.

2. Procédé suivant la revendication 1, caractérisé en ce qu'on mesure le rayonnement γ instantané à 1,26 MeV.

3. Procédé suivant la revendication 2, caractérisé en ce qu'on mesure en outre le rayonnement γ à 2,23 et 5,3 MeV.

4. Procédé suivant la revendication 1, caractérisé en ce qu'on détermine en outre le rayonnement γ retardé de produits d'activation formés.

5. Dispositif pour la réalisation du procédé suivant la revendication 1, caractérisé en ce qu'il comporte un point de mesure que l'on doit introduire dans un parcours de transport de la viande et qui comporte une source de rayons n disposée de telle sorte que les masses de viande qui défilent, soient exposées au rayonnement de cette source, ce point de mesure comportant également au moins un détecteur de rayons γ (2, 3) auquel sont raccordés un discriminateur (4) et éventuellement un calculateur (5).

6. Dispositif pour la détermination de la teneur en graisse de masses de viande suivant la revendication 1, caractérisé en ce qu'il comporte au moins un chariot de transport de viande (6) pourvu d'un tunnel ou d'un bac intérieur (9) ouvert sur ses faces frontales ce dispositif comportant également un point de mesure installé dans le parcours de transport de la masse de viande et comportant une source de neutrons (1) pénétrant dans ce bac ou ce tunnel lorsque le chariot précité passe devant le point de mesure, des détecteurs de rayons γ (2, 3) auxquels sont raccordés un analyseur (4) et éventuellement un calculateur (5), étant installés à côté ou à l'extérieur du chariot de transport.

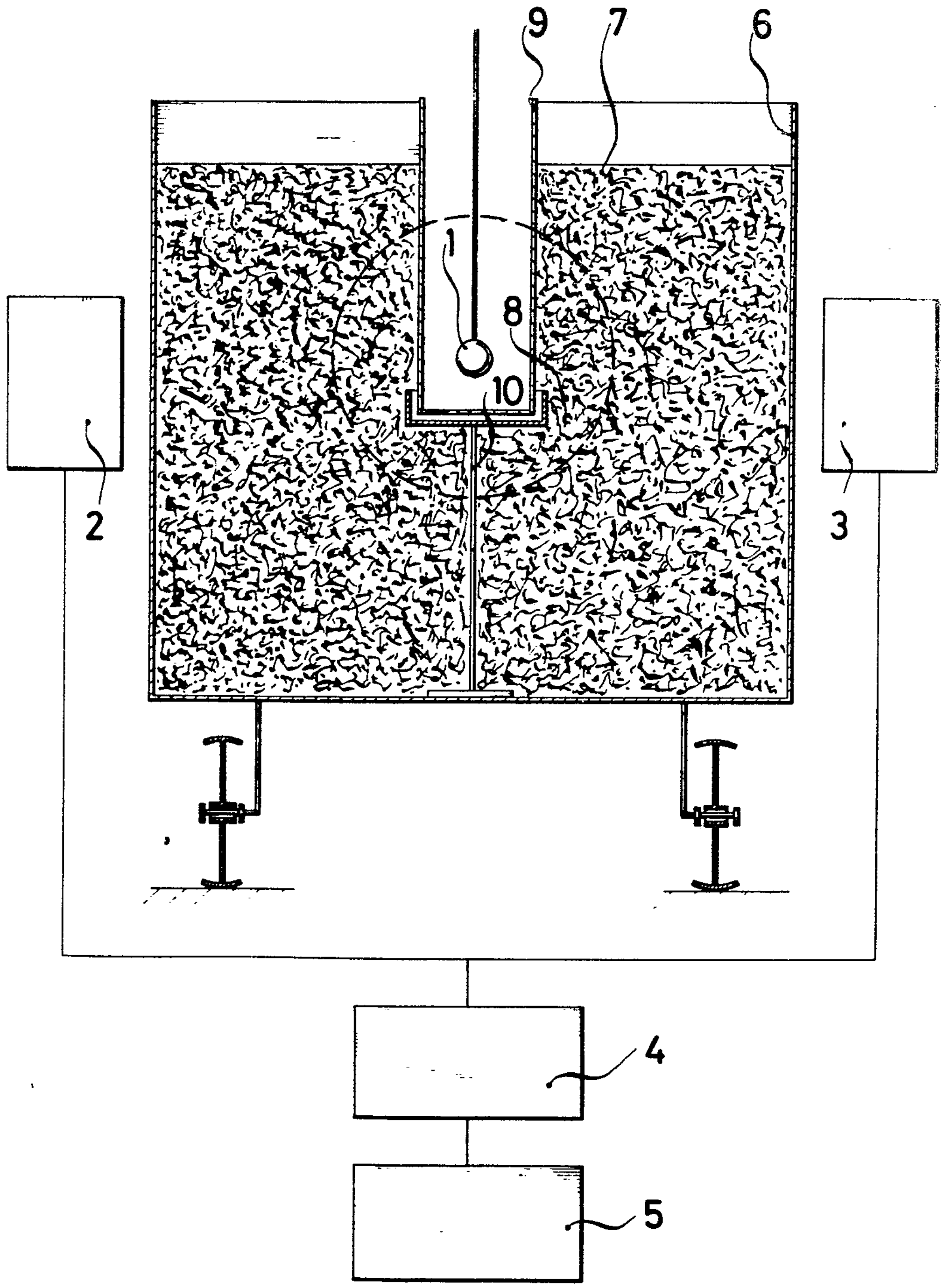
9
7
6
1
8
10
2
3
4
5